# EUROPÄISCHE PATENTSCHRIFT

(11) **EP 0 966 943 B1**
(45) Veröffentlichungstag und Bekanntmachung des Hinweises auf die Patenterteilung: **05.03.2008**
(21) Anmeldenummer: 99109055.6
(22) Anmeldetag: 07.05.1999
(51) Int. Cl.: A61G 1/00, A61G 1/04

(54) **Vorrichtung zum Transport von Kranken zwischen einem Erkrankungsort und einer medizinischen Einrichtung**
Device for transporting patients between the location of sickness and a medical institution
Dispositif pour transporter des patients entre le site de maladie et une institution médicale

(30) Priorität: 14.05.1998 DE 19821692
(43) Veröffentlichungstag der Anmeldung: 29.12.1999
(73) Patentinhaber: Airbus Deutschland GmbH, 21129 Hamburg (DE)
(72) Erfinder: Schumacher, Markus, 21614 Buxtehude (DE); Muin, Andrew, 21698 Harsefeld (DE)
(74) Vertreter: Hansmann, Dierk

(56) Entgegenhaltungen:
- WO-A-96/03955
- DE-A- 4 416 244
- DE-U- 29 714 293
- GB-A- 126 442
- US-A- 4 747 172
- US-A- 5 149 030
- US-A- 5 494 051

## Beschreibung

Die Erfindung betrifft eine Vorrichtung zum Transport von Kranken zwischen einem Erkrankungsort und einer medizinischen Einrichtung, im wesentlichen bestehend aus einer Krankentrage, die ein Rahmengestell und eine Auflagefläche aufweist, und medizinischen Versorgungsgeräten, wie Infusionspumpe, Sauerstoffgerät, Defibrillator, wobei an der Krankentrage Mittel zum Halten zumindest eines medizinischen Versorgungsgerätes vorgesehen sind und das entsprechende medizinische Versorgungsgerät als Versorgungsmodul ausgebildet ist.

Nach dem derzeitigen Stand der Technik ist es üblich, dass ein Kranker bzw. Unfallopfer vom Erkrankungsort zu einem Krankenhaus mittels eines Transportmittels, beispielsweise Notarztwagen, gebracht wird. Beim Transport des Kranken, insbesondere eines schwerkranken Patienten, auf einer Krankentrage vom und zum Transportmittel müssen häufig benötigte medizinische Geräte, wie Infusionspumpe, Sauerstoffgerät, Defibrillator, EKG usw. ebenfalls mitgeführt werden.

DE-A-4416244 zeigt eine Krankentrage, die unter anderem Hohlkörper zur Aufnahme von zusätzlichen Rettungsmitteln beinhaltet. In diese als Einschubfächer dienende Hohlkörper können Versorgungsmodule eingeschoben werden. Weiterhin ist ein Hohlraum zur Aufnahme eines Geräteträgers, vorzugsweise im Kopfteilbereich, ersichtlich. Der Geräteträger ist in den Hohlraum einschiebbar und zur Aufnahme von weiteren Versorgungsmodulen vorgesehen. Durch die Ausbildung der Aufnahmen als Hohlräume ist es von Nachteil, dass nur in den Abmessungen festgelegte Geräte in den Einschubfächem aufgenommen werden können. Ein Trennen und Umsetzen der Versorgungsmodule/Geräte ist nicht vorgesehen.

Auch WO-A-9603955 zeigt eine Krankentrage mit einer Halterung von medizinischen Geräten. Hier werden die Versorgungsmodule in Gehäuse seitlich und unterhalb der Trage eingeschoben. Mehrere Versorgungsmodule sind typischerweise nebeneinander angeordnet. Für die Rettungspersonen, die die Krankentrage tragen und somit an Kopf- und Fußseite der Trage an den Haltegriffen anfassen, ist es somit nicht möglich, während des Transportes alle, mit dem Anzeigedisplay senkrecht zur Längsrichtung der Trage ausgerichtete medizinischen Geräte zu überblicken. Auch ist ein Trennen und Umsetzen der Versorgungsmodule/Geräte nicht vorgesehen.

Aus DE A 29714293 ist eine Befestigungshalterung zur Befestigung von Geräten an Krankenbetten oder an Transportgeräten bekannt. Der abgeschrägte Bereich an den Haltehaken ermöglicht, dass an Haltestangen mit unterschiedlichen Durchmessern eine Halterung erzielt werden kann. Die Aufnahme von Geräten erfolgt auf einer horizontalen Halteplatte. Das Gerät wird mit seinen Füßen insbesondere in Aussparungen auf der Halteplatte positioniert. Spezielle Aufnahmen, die eine kompaktere Ausbildung der Halteplatte erforderlich machen würden, um beispielsweise spezielle lebensnotwendige Geräte transportieren zu können, sind dieser Druckschrift nicht zu entnehmen.

Die US-A-4747172 zeigt eine Krankentrage, an dessen Halterung ein Modulträger zum Halten von medizinischen Geräten eingerastet werden kann. Der Modulträger ist abnehmbar gestaltet, kann jedoch nicht durch eine Person in der Weise getragen werden, dass die Hände frei bleiben und damit gerade für einen lückenlosen Transport - wenn diese Person z.B. gleichzeitig die Krankentrage tragen soll und der Modulträger aber aufgrund der Wegverhältnisse nicht an der Trage befestigt bleiben kann - mit nur zwei Hilfskräften realisiert werden.

Auch aus DE-U-29714293 ist ein Gerätehalter bekannt, der an einem Krankenbett oder an anderen Transportgeräten befestigt werden kann. Ein Transport des Gerätehalters durch eine Person, die gleichzeitig auch noch die Hände zum Transport der Liege oder für Hilfsmaßnahmen am Kranken frei hat, ist nicht ersichtlich.

Der Erfindung liegt daher die Aufgabe zugrunde, eine Vorrichtung zum Transport von Kranken zu schaffen, die den Anforderungen eines modernen Rettungsdienstes gerecht wird und eine lückenlose medizinische Geräteversorgung vom Unfall- bzw. Erkrankungsort zum Krankenhaus oder Zielort ermöglicht und insbesondere das Handhaben und Umsetzen der medizinischen Geräte vereinfacht.

Diese Aufgabe wird bei einer gattungsgemäßen Vorrichtung mit den im Patentanspruch 1 genannten Maßnahmen gelöst.

Dabei ist insbesondere von Vorteil, dass es mit der Anordnung der wichtigen Versorgungsgeräte an der Krankentrage möglich ist, zwischen Aufnahme- bzw. Unfallort und dem Transportmittel und danach zwischen dem Transportmittel und dem Zielort eine lückenlose medizinische Versorgung ohne einen zusätzlichen Aufwand personeller Art zu erreichen. Mit Mitteln zum Halten der Versorgungsmodule am Rahmengestell im Bereich des Kopf- und/oder Fußendes der Krankentrage ist ein Überblick über die Anzeigeelemente der Versorgungsmodule für das tragende Personal verbessert. Mit der Ausbildung eines Modulträgers als eine separate, von der Krankentrage trennbare Einheit kann er bei Bedarf ohne Probleme an der Krankentrage angeordnet, auch wieder abgenommen und von einer Person getragen werden.

Ein erfindungsgemäßer Modulträger zum Halten von medizinischen Versorgungsgeräten ist im Patentanspruch 5 aufgeführt.

Weiterbildungen und vorteilhafte Ausgestaltungen der Vorrichtung zum Transport von Kranken sind in den Unteransprüchen 2 bis 4 angegeben.

Weiterbildungen und Ausgestaltungen des Modulträgers sind in den Unteransprüchen 6 bis 9 angegeben.

In der Zeichnung sind Ausführungsbeispiele der Erfindung dargestellt, die nachstehend anhand der Figuren 1 bis 11 näher beschrieben werden. In den Figuren sind gleiche Bauteile mit gleichen Bezugszeichen versehen.

Es zeigt
- Fig. 1: eine Vorrichtung zum Transport von Kranken in einer ersten Ausführungsform die nicht gemäß der Erfindung ausgebildet ist,
- Fig. 2: ein Rahmengestell einer Krankentrage gemäß Fig. 1,
- Fig. 3: ein an der Krankentrage angeordnetes Überwachungsdisplay als Einzelheit aus Fig. 1,
- Fig. 4: eine Krankentrage gemäß Fig. 1 mit darunter angeordneten Versorgungsmodulen,
- Fig. 5: einen Geräeschrank für Versorgungsmodule,
- Fig. 6: eine Vorrichtung zum Transport von Kranken in einer zweiten Ausführungsform gemäß der Erfindung,
- Fign. 7, 7A, 7B: ein Modulträger als Einzelheit aus Fig. 6,
- Fig. 8: ein Modulträger mit darauf angeordneten Versorgungsmodulen,
- Fig. 9 und 10: Ausgestaltungen der Halterung eines Versorgungsmoduls und
- Fig. 11: eine Krankentrage mit Kombination der Ausführungsformen gemäß Fig. 1 und Fig. 6.

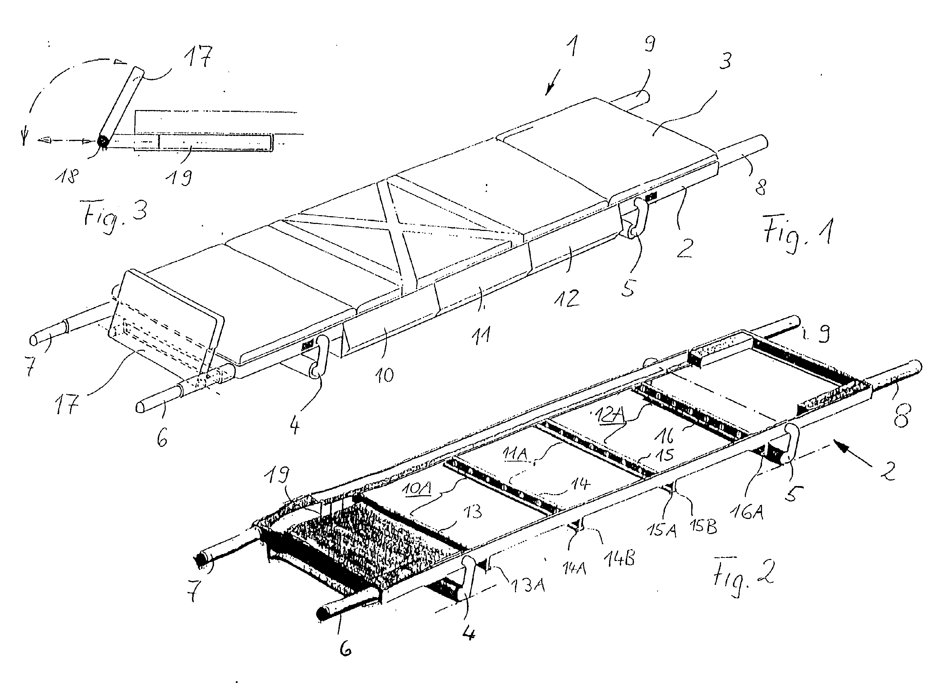

In der Fig. 1 ist eine Vorrichtung zum Transport von Kranken ersichtlich in einer ersten Ausführungsform, die nicht gemäß der Erfindung ausgebildet ist. Die Vorrichtung zum Transport von Kranken weist eine Krankentrage 1 auf, die im wesentlichen besteht aus einem Rahmengestell 2 und einer Auflagefläche 3. Das Rahmengestell 2 weist bügelförmige Fußstützen 4 und 5 sowie Tragegriffe 6, 7, 8 und 9 auf. Die Tragegriffe 6, 7, 8 und 9 können teleskopartig in das Rahmengestell 2 einschiebbar sein und entsprechen im eingeschobenen Zustand somit insbesondere beim Transport in den vorgesehenen Transportmitteln wie Notarztwagen, Hubschrauber oder Flugzeug den zumeist beengten Platzverhältnissen. Unterhalb der Krankentrage 1, vorzugsweise im Bereich zwischen den Fußstützen 4 und 5, sind Versorgungsmodule 10, 11 und 12 vorgesehen, die jeweils ein medizinisches Versorgungsgerät wie beispielsweise Infusionspumpe, Sauerstoffgerät oder Defibrillator aufnehmen. Mittels der Fußstützen 4 und 5 ist zwischen Rahmengestell 2 und Abstellfläche der Krankentrage 1 eine solche Höhe realisiert, dass die Versorgungsmodule 10, 11 oder 12 auch bei abgestellter Krankentrage 1 ausreichend Platz haben. Zum Halten der Versorgungsmodule 10, 11 und 12 sind unterhalb der Krankentrage 1 am Rahmengestell 2 Halteschienen 13, 14, 15, 16 vorgesehen, die senkrecht zur Längsachse des Rahmengestells 2 verlaufen und jeweils Modulaufnahmen 10A, 11A, 12A bilden (siehe Fig. 2).

Das Rahmengestell 2 ist in Fig. 2 ersichtlich, welches nicht gemäß der Erfindung ausgebildet ist.. Die äußeren Halteschienen 13 und 16 weisen jeweils eine Eingriffsleiste 13A bzw. 16A auf, die in eine Nut am Versorgungsmodul eingreifen kann und dieses Modul dann schubladenartig unterhalb der Krankentrage eingeschoben und gehalten werden kann. Die mittleren Halteschienen 14 und 15 können vorzugsweise auch zwei Eingriffsleisten 14A und 14B sowie 15A und 15B aufweisen, sodass mehrere Versorgungsmodule hintereinander angeordnet werden können. Die Versorgungsmodule können je nach Abmessung auch von beiden Seiten der Krankentrage 1 aufgenommen werden, sodass auch zwei Module nebeneinander angeordnet sind. Die Versorgungsmodule 10, 11 und 12, die üblicherweise mit elektrischer Energie versorgt werden müssen, können über Kontakte an den Halteschienen 13, 14, 15, 16 über die Krankentrage 1 mit einer zentralen Energieversorgung, beispielsweise das Bordnetz im Transportmittel, gekoppelt werden. Während des Transports der Trage von und zum Transportmittel ist eine Energieversorgung separat in jedem Versorgungsmodul mittels Akkumulatoren vorgesehen. Eine Kopplung über Kontakte an den Halteschienen zwischen Trage und Versorgungsmodul ist auch zur Datenübertragung nutzbar. So kann am Kopf- oder Fußende der Trage ein Überwachungsdisplay 17 angeordnet sein, der zur Anzeige wesentlicher medizinischer Daten vorgesehen ist, die von den Versorgungsgeräten übermittelt werden. Ein Sanitäter, der die Krankentrage an Kopf- oder Fußende trägt, kann somit zugleich Überwachungsfunktionen ausüben. Auch ist möglich, daß neben einer direkten Bedienung der Versorgungsgeräte in den Versorgungsmodulen 10, 11 und 12 eine Bedienung über Schaltelemente am Überwachungsdisplay 17 erfolgen kann.

In der Fig. 3 ist das Überwachungsdisplay 17 der nicht erfindungsgemäßen ersten Ausführungsform als Einzelheit dargestellt. Es ist um einen Drehpunkt 18 schwenkbar. In abgeklappter Position, d.h. das Überwachungsdisplay 17 liegt parallel und unterhalb der Auflagenflächeebene, kann das Display 17 in eine kastenförmige Displayaufnahme 19 eingeschoben werden. Somit ist es bei Nichtgebrauch auch so ablegbar, daß es nicht die Versorgung eines Kranken behindert:

Aufgrund der Anordnung der wichtigen Versorgungsgeräte an der Krankentrage ist es möglich, zwischen Aufnahme- bzw. Unfallort und dem Transportmittel und danach zwischen dem Transportmittel und dem Zielort eine lückenlose medizinische Versorgung ohne einen zusätzlichen Aufwand personeller Art zu erbringen.

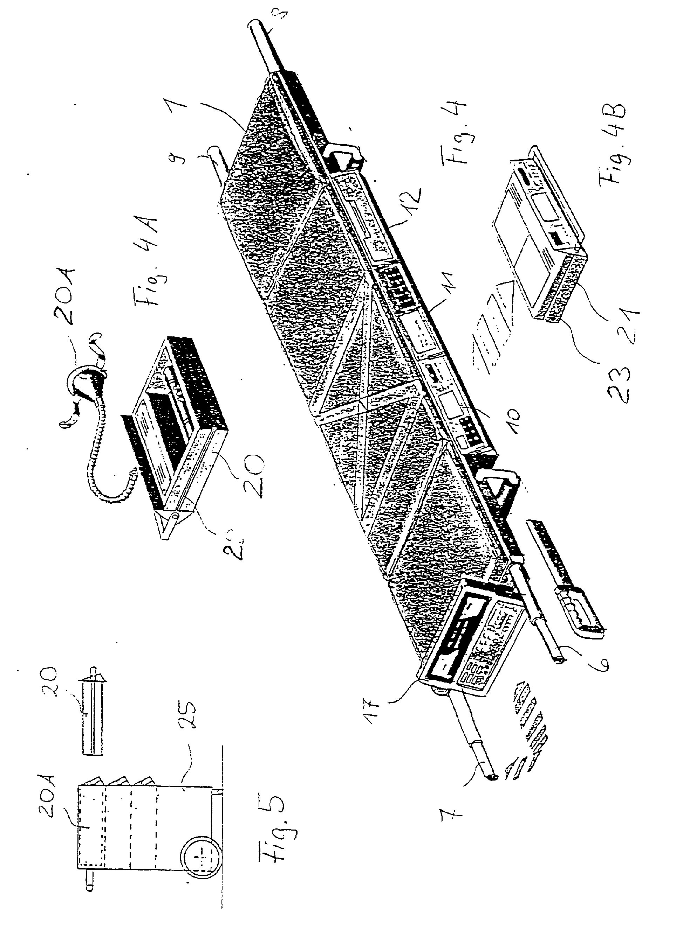

In Fig. 4 ist eine Krankentrage 1 gemäß der ersten, nicht erfindungsgemäßen Ausführungsform in perspektivischer Darstellung gezeigt. Versorgungsmodule 10, 11 und 12, die die benötigten Versorgungsgeräte enthalten, sind unterhalb der Krankentrage 1 angeordnet. Die Versorgungsmodule - als separate Bauteile - sind austauschbar. Somit können entsprechend des jeweiligen Notfalleinsatzes die jeweils notwendigen Versorgungsgeräte zur Verfügung gestellt werden. Ersichtlich in Fig. 4A und 4B ist jeweils ein Versorgungsmodul 20 bzw. 21. Das Versorgungsmodul 20 ist für die Sauerstoffversorgung vorgesehen. Es kann eine Sauerstoffmaske 20A angeschlossen werden. Das Versorgungsmodul 21 enthält z. B. das Versorgungsgerät Defibrillator. Die Versorgungsmodule sind mit seitlichen Nuten mit Kontaktschienen 22 bzw. 23 versehen, damit nach dem Einschieben auf den dafür vorgesehenen Halteschienen eine Datenverbindung zur Krankentrage 1 hergestellt ist und über das Überwachungsdisplay 17 die Gerätefunktionen überwacht werden können. Über die Kontaktschienen 22 bzw. 23 ist auch die Zuführung der elektrischen Energie zu den Versorgungsgeräten realisierbar.

In Fig. 5 ist ein nicht erfindungsgemäßer Geräteschrank 25 für Versorgungsmodule dargestellt. In diesem Geräteschrank 25, der als eine mobile, verfahrbare Einheit ausgebildet ist, können die Versorgungsmodule (beispielhaft Versorgungsmodul 20) in übereinander angeordneten Modulaufnahmen (beispielhaft 20A) untergebracht werden. Wenn ein Patient von der Krankentrage 1 zur stationären Behandlung in einem Krankenhaus auf ein dort vorhandenes Krankenbett umgelegt werden soll, können die bereits am Patienten angeschlossenen Versorgungsgeräte im entsprechenden Versorgungsmodul aus der Krankentrage 1 entnommen werden und in dem Geräteschrank 25 untergebracht werden. Hierbei kann auch eine zentrale Energieversorgung der einzelnen Versorgungsmodule über Kontakte in diesem Geräteschrank 25 erfolgen oder auch eine Datenübertragung zur überwachung der Körperfunktionen über eine zentrale Einheit realisiert werden. Mit dem Einsatz eines solchen Geräteschrankes 25, der die Versorgungsmodule am Krankenbett des Patienten aufnimmt, ist eine lükenlose Patientenversorgung gewährleistet.

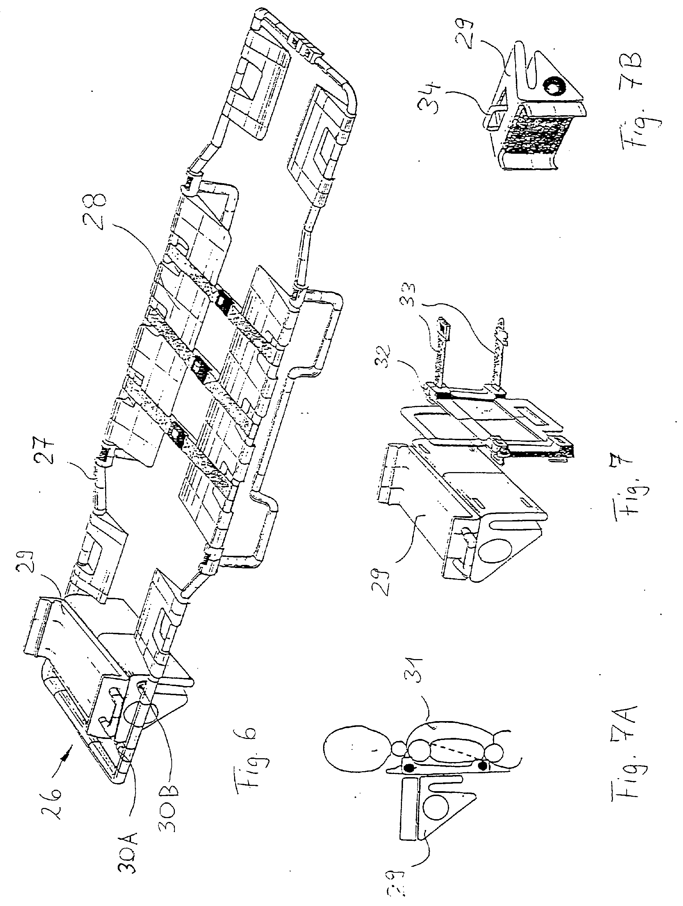

Eine zweite, erfindungsgemäße Ausführungsform einer Vorrichtung zum Transport von Kranken ist in den Fign. 6 ff. gezeigt.
Die Fig. 6 zeigt eine Krankentrage 26, die im wesentlichen ein Rahmengestell 27 und eine Auflagefläche 28 aufweist. Am Kopf- bzw. Fußende der Krankentrage 26 sind Mittel zum Halten eines Modulträgers 29 vorgesehen. Der Modulträger 29 ist als separate Einheit ausgebildet, der Versorgungsmodule zur Versorgung eines Patienten auf der Krankentrage 26 aufnehmen kann. Dafür sind an dem Rahmengestell 27 im Kopf- oder Fußbereich zwei Transportstangen 30A und 30B angeordnet. Der Modulträger 29 kann auch von der Krankentrage 26 abgenommen werden und von einer Begleitperson der Krankentrage 26 gehalten werden.
In den Fign. 7 und 7B sind dazu Trageinrichtungen am Modulträger 29 ersichtlich. Die Fig. 7 zeigt den Modulträger 29, an den eine Trageinrichtung 32 angekoppelt werden kann. Die Trageinrichtung 32 weist Tragriemen 33 auf, damit ein rucksackähnlicher Transport durch eine Begleitperson 31 der Krankentrage 26 (siehe Fig. 7A) erfolgen kann. Die Trageinrichtung 32 kann vom Modulträger 29 abnehmbar gestaltet sein, In Fig. 7B ist eine weitere Tragemöglichkeit des Modulträgers 29 gezeigt. Dafür ist an einer Seite des Modulträgers 29 ein Handgriff 34 vorgesehen. Beispielsweise bei nur kurzen Strecken kann die Begleitperson den Modulträger 29 mit der Hand tragen.

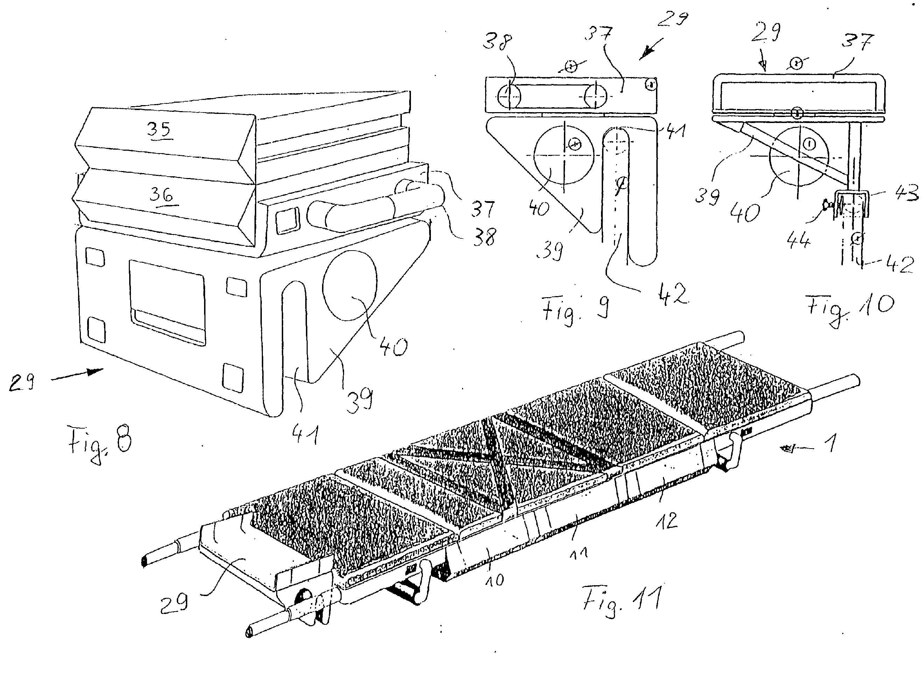

In der Fig. 8 ist die Einzelheit des Modulträgers 29 perspektivisch dargestellt. Der Modulträger 29 weist ein Traggestell 39 und eine Modulplattform 37 auf, die zur Aufnahme von Versorgungsmoduln 35 und 36 dient. Die Modulplattform 37 ist vorzugsweise drehbar gestaltet, um eine optimale Ablesemöglichkeit der Displayanzeigen an den Geräten der Versorgungsmodule 35 und 36 zu erreichen. Die Versorgungsmodule 35 und 36 sind so gestaltet bzw. weisen ein solches Gehäuse auf, daß mehrere Module übereinander angeordnet werden können und in dieser Position auch gehalten werden. Zum Tragen des Modulträgers 29 mit darauf angeordneten Versorgungsmodulen 35 und 36 sind an der Modulplattform Handgriffe 38 vorgesehen. Ein Anwendungsfall ist beispielsweise wenn ein Patient von der Krankentrage 26 in ein Krankenbett umgelegt wird und die am Patienten angeschlossenen Geräte ebenfalls am Krankenbett aufgebaut werden sollen. Das Traggestell 39 dient zur Befestigung und Halterung der Modulplattform 37 an der Krankentrage 26 bzw. einem Krankenbett und muß deshalb so dimensioniert sein, daß mehrere Versorgungsmodule, z.B. Versorgungsmodul 35 und 36 ausreichend stabil gehalten werden. Das Traggestell 39 ist somit ausreichend groß, um gleichzeitig auch ein wichtiges, fast immer benötigtes Versorgungsgerät, wie ein Sauerstoffgerät 40 mit Sauerstoffmaske aufzunehmen. Es ist zweckmäßig, das Traggestell 39 mit einem Gehäuse zu versehen. Damit ist Stauraum für das Sauerstoffgerät 40 und die Sauerstoffmaske geschaffen. Das Traggestell weist weiterhin eine über die Breite des Modulträgers 29 verlaufende Aussparung 41 auf, die eine u-förmige Aufnahme im Traggestell 39 bildet und so in einem Fall an der Krankentrage 26 an der Transportstange 30B und im anderen Fall bei der Befestigung des Modulträgers 29 am Krankenbett an einem Rand des Bettgestells adaptierbar ist.

In den Fign. 9 und 10 sind unterschiedliche Befestigungsmöglichkeiten des Modulträgers 29 aufgeführt. In Fig. 9 ist die bereits in Fig. 8 beschriebene Halterung durch die Aussparung 41 am Traggestell 39 gezeigt, die ein Krankenbettgestell 42 aufnehmen kann. Aufgrund der tiefen Aussparung 41 können die Gewichtskräfte, die von der Modulplattform 37 und den Versorgungsmodulen auf das Traggestell 39 wirken, gleichzeitig als Haltekraft für den gesamten Modulträger eingesetzt werden und eine zusätzliche Befestigung des Traggestells 39 am Bettrand 42 ist nicht notwendig.

In Fig. 10 ist eine Ausgestaltung des Traggestells 39 gezeigt, die eine u-profilartige Randaufnahme 43 enthält. Hier ist, um eine ausreichende Festigkeit der Halterung zu erreichen, ein zusätzliches Spannelement 44 notwendig.

Die Fig. 11 zeigt eine Vorrichtung zum Transport von Kranken in einer Kombination der ersten und zweiten Ausführungsform. An der Krankentrage 1 sind unterhalb des Rahmengestells 2 Versorgungsmodule 10, 11 oder 12 angeordnet. Diese Versorgungsmodule können neben Versorgungsgeräten auch einen Notarztkoffer oder Verbandsmaterial beinhalten. Am Kopf- oder Fußende der Krankentrage 1 ist ein Modulträger 29 angeordnet.

## Patentansprüche

1. Vorrichtung zum Transport von Kranken zwischen einem Erkrankungsort und einer medizinischen Einrichtung, im wesentlichen bestehend aus einer Krankentrage (1), die ein Rahmengestell (2) und eine Auflagefläche (3) aufweist, und medizinischen Versorgungsgeräten, wie Infusionspumpe, Sauerstoffgerät, Defibrillator, wobei an der Krankentrage (1) Mittel zum Halten zumindest eines medizinischen Versorgungsgerätes vorgesehen sind und das entsprechende medizinische Versorgungsgerät als Versorgungsmodul ausgebildet ist,
**dadurch gekennzeichnet, dass** die Mittel zum Halten der Versorgungsmodule am Rahmengestell (27) am Kopf- und/oder Fußende der Krankentrage (26) angeordnet sind und einen Modulträger (29) bilden, wobei der Modulträger (29) als eine separate, von der Krankentrage (1) trennbare Einheit ausgebildet ist, das Rahmengestell (27) der Krankentrage (26) zumindest eine Transportstange (30A, 30B) aufweist und der Modulträger (29) an der Transportstange (30A, 30B) adaptierbar ist sowie eine für den rucksackähnlichen Transport durch eine Begleitperson geeignete Trageinrichtung (32) mit Tragriemen (32) oder Traggurten aufweist.

2. Vorrichtung nach Anspruch 1,
**dadurch gekennzeichnet, dass**
am Rahmengestell (2) der Krankentrage (1) in Sichtbereich einer Begleitperson ein Überwachungsdisplay (17) als Überwachungseinrichtung angeordnet ist.

3. Vorrichtung nach einem der Ansprüche 1 oder 2,
**dadurch gekennzeichnet, dass**
die Trageinrichtung (32) vom Modulträger (29) abnehmbar gestaltet ist.

4. Vorrichtung nach einem der Ansprüche 1 bis 3,
**dadurch gekennzeichnet, dass**
der Modulträger (29) einen Handgriff (34) aufweist.

5. Modulträger zum Halten von medizinischen Versorgungsgeräten, der mit der Vorrichtung zum Transport von Kranken nach Anspruch 1 zusammenwirkt,
**dadurch gekennzeichnet, dass**
der Modulträger (29) als separate Einheit ausgebildet ist, der ein Traggestell (39) und eine Modulplattform (37) aufweist und eine Aufnahme (41) am Traggestell (39) gebildet ist zur Adaption an der Transportstange (30A, 30B) des Rahmengestells (27) oder an einem Krankenbett (42) sowie eine für den rucksackähnlichen Transport durch eine Begleitperson geeignete Trageinrichtung (32) mit Tragriemen (32) oder Traggurten angeordnet ist.

6. Modulträger nach Anspruch 5,
**dadurch gekennzeichnet; dass**
die Trageinrichtung (32) vom Modulträger (29) abnehmbar gestaltet ist.

7. Modulträger nach einem der Ansprüche 5 oder 6,
**dadurch gekennzeichnet, dass**
der Modulträger (29) mit mindestens einem Handgriff (34) versehen ist.

8. Modulträger nach einem der Ansprüche 5 bis 7,
**dadurch gekennzeichnet, dass**
das Traggestell (39) ein Gehäuse zur Aufnahme eines Sauerstoffgerätes (40) sowie Stauraum für eine Sauerstoffmaske aufweist.

9. Modulträger nach einem der Ansprüche 5 bis 8,
**adurch gekennzeichnet**, **dass**
die Modulplattform (37) drehbar ist.

## Claims

1. Device for transporting patients between the place at which they were taken ill and a medical establishment, said device essentially consisting of a stretcher (1), which has a frame (2) and a supporting surface (3), and of medical care appliances such as an infusion pump, oxygen apparatus and defibrillator, wherein means for holding at least one medical care appliance are provided on said stretcher (1) and the corresponding medical care appliance is constructed as a care module,
**characterised in that** the means for holding the care module on the frame (27) are disposed at the head end and/or foot end of the stretcher (26) and form a module-carrier (29), wherein said module-carrier (29) is constructed as a separate unit which can be detached from the stretcher (1), the frame (27) of the stretcher (26) has at least one transporting bar (30A, 30B) and the module-carrier (29) can be fitted to said transporting bar (30A, 30B) and also has a carrying arrangement (32) which has carrying straps (32) or carrying belts and is suitable for rucksack-like transport by an attendant.

2. Device according to claim 1,
**characterised in that**
a monitoring display (17) in the form of a monitoring apparatus is disposed on the frame (2) of the stretcher (1) within the field of vision of an attendant.

3. Device according to one of claims 1 or 2,
**characterised in that** the carrying arrangement (32) is designed so as to be removable from the module-carrier (29).

4. Device according to one of claims 1 to 3,
**characterised in that**
the module-carrier (29) has a handle (34).

5. Module-carrier for holding medical care appliances which interacts with the device for transporting patients according to claim 1,
**characterised in that**
the module-carrier (29) is constructed as a separate unit and has a carrying frame (39) and a module platform (37), and there is formed, on said carrying frame (39), a receptacle (41) for fitting to the transporting bar (30A, 30B) of the frame (27) or to a patient's bed (42), and a carrying arrangement (32) is also provided, which has carrying straps (32) or carrying belts and is suitable for rucksack-like transport by an attendant.

6. Module-carrier according to claim 5,
**characterised in that**
the carrying arrangement (32) is designed so as to be removable from the module-carrier (29).

7. Module-carrier according to one of claims 5 or 6,
**characterised in that**
the module-carrier (29) is provided with at least one handle (34).

8. Module-carrier according to one of claims 5 to 7,
**characterised in that**
the carrying frame (39) has a housing for receiving an oxygen apparatus (40) and also stowage space for an oxygen mask.

9. Module-carrier according to one of claims 5 to 8,
**characterised in that**
the module platform (37) is rotatable.

## Revendications

1. Dispositif de transport de malades entre le lieu où survient la maladie et une installation de soins médicaux, constitué essentiellement d'une civière (1), qui présente un cadre (2) et une surface de couchage (3), et d'appareils de soins médicaux, tels qu'une pompe à perfusion, un inhalateur à oxygène ou un défibrillateur, des moyens de support d'au moins un appareil de soins médicaux étant prévus sur la civière (1), et l'appareil de soins médicaux correspondant étant réalisé sous forme de module de soins,
**caractérisé par le fait que** les moyens de support des modules de soins sont disposés sur le cadre (27), à la tête et/ou au pied de la civière (26), et forment un support de module (29), ledit support de module (29) étant réalisé sous forme d'unité distincte, pouvant être séparée de la civière (1), le cadre (27) de la civière (26) présentant au moins une barre de transport (30A, 30B), et le support de module (29) pouvant être adapté sur la barre de transport (30A, 30B) et présentant un dispositif de support (32) doté de sangles (33) ou de bretelles et adapté au transport par un accompagnateur, à la manière d'un sac à dos.

2. Dispositif selon la revendication 1, **caractérisé par le fait qu'**un écran de surveillance (17) est installé en tant que moyen de surveillance sur le cadre (2) de la civière (1), dans le champ de vision d'un accompagnateur.

3. Dispositif selon une des revendications 1 ou 2, **caractérisé par le fait que** le dispositif de support (32) est conçu de manière à pouvoir être retiré du support de module (29).

4. Dispositif selon une des revendications 1 à 3, **caractérisé par le fait que** le support de module (29) présente une poignée (34).

5. Support de module destiné à tenir des appareils de soins médicaux et coopérant avec le dispositif de transport de malades selon la revendication 1, **caractérisé par le fait que** le support de module (29) est réalisé sous forme d'unité distincte, qui présente un bâti de support (39) et une plateforme de module (37), et qu'un logement (41) est ménagé sur le bâti de support (39), en vue d'une adaptation sur la barre de transport (30A, 30B) du cadre (27) ou sur un lit de malade (42), et qu'il, est prévu un dispositif de support (32) doté de sangles (33) ou de bretelles et adapté au transport par un accompagnateur, à la manière d'un sac à dos.

6. Support de module selon la revendication 5, **caractérisé par le fait que** le dispositif de support (32) est conçu de manière à pouvoir être retiré du support de module (29).

7. Support de module selon une des revendications 5 ou 6, **caractérisé par le fait que** le support de module (29) est pourvu d'au moins une poignée (34).

8. Support de module selon une des revendications 5 à 7, **caractérisé par le fait que** le bâti de support (39) présente un boîtier destiné à recevoir un inhalateur à oxygène (40), ainsi qu'un espace de rangement pour un masque à oxygène.

9. Support de module selon une des revendications 5 à 8, **caractérisé par le fait que** la plateforme de module (37) peut tourner.
